# EUROPEAN PATENT SPECIFICATION

(11) **EP 3 946 265 B1**
(45) Date of publication and mention of the grant of the patent: **04.12.2024**
(21) Application number: 20716120.9
(22) Date of filing: 02.04.2020
(51) Int. Cl.: A61K 9/08, A61K 47/02, A61K 47/12, A61K 47/36, A61P 29/00, A61P 31/04, A61K 31/573, A61K 31/5383

(54) **OTOLOGIC COMPOSITIONS**
OTOLOGISCHE ZUSAMMENSETZUNGEN
COMPOSITIONS OTOLOGIQUES

(30) Priority: 05.04.2019 IT 201900005280
(43) Date of publication of application: 09.02.2022
(73) Proprietor: NTC S.r.l., 20124 Milano (IT)
(72) Inventor: MARCELLONI, Luciano, 20124 Milano (MI) (IT); BERTOCCHI, Federico, 20124 Milano (MI) (IT); CARNOVALI, Marino, 20124 Milano (MI) (IT)
(74) Representative: Benedetto, Marco
(86) International application number: PCT/IB2020/053136
(87) International publication number: WO 2020/202056

(56) References cited:
- EP-A1- 3 216 451
- CN-A- 101 199 855
- CN-A- 105 560 252
- RU-C1- 2 642 617
- US-A1- 2001 049 366
- US-A1- 2013 178 801

## Description

The present invention regards an otological composition in aqueous solution, for use in a method for the treatment of bacterial ear infections, preferably for the treatment of acute otitis externa (AOE) and acute otitis media (AOM).

Acute otitis externa (AOE) is one of the most common infections observed by physicians, with individual variations depending on the age of the subject, geographical area and seasonality. The incidence of infection in paediatric subjects of age comprised from 5 to 14 years is still very high and it is in the order of 10% (*Lifetime incidence*). Acute otitis externa (AOE) is defined as a widespread inflammation of the external auditory canal, which may also involve the pinna or the tympanic membrane. A diagnosis of widespread AOE requires a rapid onset (usually within 48 hours) of symptoms and signs of inflammation of the auditory canal in the last 3 weeks. There is consensus in the published literature according to which almost all (98%) of AOE is of bacterial origin. The most common pathogens are *Pseudomonas aeruginosa* (prevalence of 20% -60%) and *Staphylococcus aureus* (prevalence of 10% -70%), which often occur as polymorphic infection. Other pathogens are mainly Gram-negative organisms (other than *P. aeruginosa*), each of which causes no more than 2% to 3% of cases in large clinical series. Common predisposing factors for AOE are moisture or prolonged exposure to water, hot temperature, dermatological conditions, trauma from anatomical anomalies or external devices, and otorrhea caused by the middle ear disease.

Otitis media is a group of inflammations or infections of the middle ear, anatomical cavity found inside the external auditory canal, between the tympanic membrane and the inner ear. Acute otitis media (AOM) is a common type of otitis media in infants and young children. In the first three years of life almost all children experience at least one episode of AOM, and about 50% of children are subject to "recurrent" episodes of AOM (at least four episodes of AOM in one year, or at least three episodes of AOM in six months) [Teele DW, Et al. J Infect Dis 1989].

AOM is a bacterial disease caused by *Streptococcus pneumoniae,* non-typable *Haemophilus influenzae* (NTHi), Moraxella *catarrhalis* and *Streptococcus pyogenes* [Ngo CC, et Al. PLoS One 2016].

The tympanic membrane remains intact in most episodes of AOM, although the membrane in these cases becomes swollen and hyperemic. Nevertheless, in a minority of episodes of AOM, the tympanic membrane undergoes spontaneous perforation (STMP; *spontaneous tympanic membrane perforation*). STMP is caused by an accumulation of pus in the (closed) cavity of the middle ear, where such accumulation exerts increasing pressure on the blood vessels of the tympanic membrane, up to perforating the latter [Berger G. J Laryngol Otol 1989].

The wholeness of the tympanic membrane is restored spontaneously within a few days from perforation, hence STMP has in the past been considered a mild complication of acute otitis media. However, more recently STMP has been reclassified as a serious complication, regarding which it is recommended to take systemic antibiotics to prevent further clinical problems [Marchisio P. et al. Int J Pediatr Otorhinolaryngol 2010].

Therefore, AOE, AOM and AOM with STMP are currently cured with antibiotic treatments, or with antibiotic and anti-inflammatory treatments, sometimes combined with pain-relieving substances.

A drug called Cilodex^{®} which combines the anti-inflammatory steroid dexamethasone with the antibiotic ciprofloxacin in a single suspension for topical otological use already exists on the market. Cilodex^{®} is recommended for the treatment of acute infections of the middle ear (otitis media) in patients with otological drainage tubes (tympanostomy tubes or "Grommets") or for the treatment of an infection of the external ear (acute otitis externa).

Another pharmacological suspension called Tobradex^{®} which contains micronised dexamethasone and tobramycin, also exists on the market. Tobradex^{®} is recommended for the treatment of the inflammation of the external auditory canal and the middle ear when the use of a corticosteroid is deemed necessary in the presence of infection caused by tobramycin-sensitive bacteria, or when there is a risk of infection.

However, it should be noted that both known products (Cilodex^{®} and Tobradex^{®}) reveal some drawbacks. In particular, in order to preserve the stability - and therefore the effectiveness - of the Cilodex^{®} and Tobradex^{®} suspensions over time, such suspensions must be kept under strict temperature limitations: as concerns Tobradex^{®}, the storage temperatures should not be lower than the freezing temperature of the suspension, and should not exceed 25°C. As concerns Cilodex^{®}, the product should be stored at temperatures higher than the freezing temperature of the suspension.

As a result, Cilodex^{®} and Tobradex^{®} products undergo degradation, alteration or become ineffective when exposed to too cold or too hot temperatures (Tobradex^{®} only). There therefore arises the problem lying in the fact that the distribution of such products in specific climatic zones (for example, in cold climates, or in zones IVa or IVb according to the World Health Organization guidelines) is subject to severe restrictions, in the absence of keen control over storage temperatures.

The prior document US 2001/0049366 A1 describes a formulation for topical use in the anti-inflammatory treatment of the eye, ear, nose or skin, wherein said formulation comprises a fluoroquinolone antibiotic such as levofloxacin and dexamethasone in a poorly water-soluble form, preferably dexamethasone alcohol or dexamethasone acetate, together with a dissolving agent. Prior document US 2001/0049366 A1 excludes the use of dexamethasone phosphate because it is a water-soluble form of dexamethasone.

Dexamethasone phosphate is a form explicitly excluded in US 2001/0049366 A1. The formulations suggested by US 2001/0049366 A1 comprise dexamethasone alcohol or dexamethasone acetate and a dexamethasone dissolving agent such as a vitamin E tocopheryl or tocopherol derivative. Although the prior document US 2001/0049366 A1 mentions a formulation in solution, such formulation is a suspension or an emulsion of dexamethasone alcohol or dexamethasone acetate in water obtained with the aid of said dissolving agent. Furthermore, said prior document does not mention a treatment of bacterial ear infections by means of said formulation, but it solely and exclusively refers to an anti-inflammatory treatment.

On the other hand, document RU 2642617 C1 regards a non-aqueous solution composition for otological use in the veterinary industry for dogs, cats and rabbits for the treatment of chronic and acute otitis caused by parasites, fungi and bacteria. Said non-aqueous solution composition comprises (for instance see Example 1) levofloxacin hemihydrate, dexamethasone sodium phosphate and an emulsifier, in particular TWEEN-80. The composition of the prior document RU 2642617 C1 is a non-aqueous solution of PEG-400, and such document does not specify the pH value of said non-aqueous solution composition.

The need is therefore felt to be able to have an antibiotic and anti-inflammatory composition with improved temperature stability, in order to allow a greater effectiveness thereof and a wider distribution in climatic zones which are difficult to reach for the known suspensions up to date.

After a long and intense research and development activity, the Applicant developed an otologic aqueous solution capable of providing an adequate response to the existing limits, drawbacks and problems.

Forming an object of the present invention is an otologic composition in solution having the characteristics as defined in the attached claims.

Preferred embodiments of the present invention will be described in the following detailed description by way of non-limiting example of the scope of protection of the present invention, with reference to the attached drawings which show:
- Figure 1 shows the structural formula of levofloxacin;
- Figure 2 shows the structural formula of dexamethasone disodium phosphate.

The composition subject of the present invention is for use in a method for the treatment of otological bacterial infections caused by levofloxacin-sensitive bacteria.

In one embodiment, this otological composition in aqueous solution, for use in a method of treatment of bacterial ear infections, for use in a method of treatment of acute otitis externa (AOE) finds a valid application when the otological bacterial infections are caused, derived or originated preferably from *Streptococcus pyogenes, Staphylococcus aureus, Staphylococcus epidermidis, Staphylococcus haemolyticus, Pseudomonas aeruginosa, Enterococcus faecalis* and *Enterobacter cloacae.*

In one embodiment, this otological composition in aqueous solution, for use in a method of treatment of bacterial ear infections, for use in a method of treatment of acute otitis media (AOM) finds a valid application when the otological bacterial infections are caused, derived or originated preferably by *Streptococcus pneumoniae, Haemophilus influenzae* and *Moraxella catarrhalis.*

Preferably, the composition subject of the present invention is for use in a method for the treatment of otological bacterial infections in subjects of any age, such as paediatric, adult and elderly subjects.

Even more preferably, the composition subject of the present invention is for use in a method for the treatment of acute otitis externa or acute otitis media, preferably in subjects with spontaneous perforation of the tympanic membrane, more preferably to replace a similar systemic oral treatment.

According to an even more preferred embodiment, the composition subject of the present invention is for topical otological use.

Forming an object of the present invention is an otologic composition in solution as claimed, wherein the composition comprises (i) a mixture in solution and, optionally, (ii) one or more pharmaceutical grade additives and/or excipients.

The mixture in solution (i) comprises or, alternatively, consists of an antibiotic (i.a) having a reduced bacterial resistance, wherein the antibiotic is levofloxacin or a salt, hydrate or hemihydrate thereof, combined or associated with a corticosteroid (i.b), wherein the corticosteroid is dexamethasone sodium phosphate ester or dexamethasone disodium phosphate ester.

Advantageously, the composition subject of the present invention is characterised by a high temperature stability and, furthermore, it has the ability not to deteriorate even if stored at temperatures above 20°C room temperature, such as for example 30°C or 35°C or 40°C for a period of time comprised from 6 to 18 months. This surprising stability can also be obtained at temperatures below freezing temperature, for example at a temperature of - 20°C. At temperatures below freezing, this surprising stability is maintained even after repeated freezing and thawing cycles. For this reason, the composition of the present invention is devoid of surfactant and suspending substances, and, as such, it is devoid of TWEEN-80 (emulsifier) and devoid of PEG-400. Furthermore, said composition may also be devoid of a dissolving agent such as for example a vitamin E tocopheryl or tocopherol derivative.

The Applicant found it useful to provide a composition in the form of ear drops which associates two active components, in the field of treatments for use to prevent, reduce or eliminate bacterial infections. In the mixture contained in the composition, one of the two active components is levofloxacin or a salt, hydrate or hemihydrate thereof, which revealed a lower environmental dispersion in past years (reduced induction of bacterial resistance) than other antibiotics such as tobramycin.

Levofloxacin is considered to be one of the antibiotics with lower bacterial resistance. The levofloxacin molecule shows one of the lowest levels of antibiotic resistance.

Levofloxacin (CAS No: 100986-85-4) is a third-generation synthetic fluoroquinolone antibacterial agent that inhibits the supercoiling activity of the DNA bacterial gyrase, blocking DNA replication. This is the isomer (L) of ofloxacin. Levofloxacin appears as a light white-yellowish-yellow-white crystal or crystalline powder. It is poorly soluble in water and has a molecular weight of 361.4 g/mol.

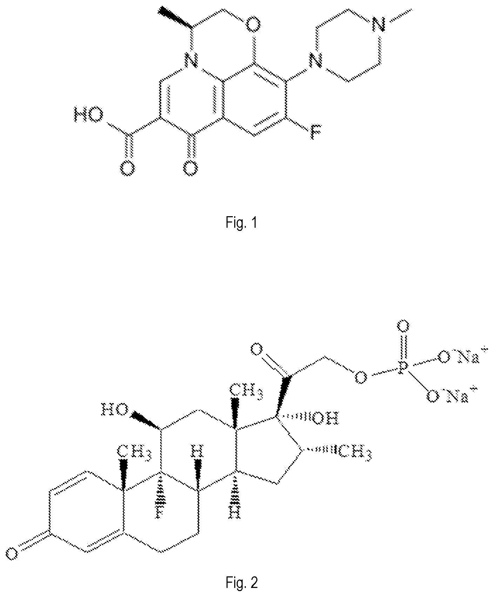

Levofloxacin, whose formula is shown in Figure 1, is more soluble in water than ofloxacin, norfloxacin or ciprofloxacin at neutral pH (Raizman et al., 2002; Koch et al., 2005). Nevertheless, it has been documented that the water solubility of levofloxacin is 1.85% after 13 weeks of mixing (Robertson et al., 2005).

Dexamethasone is a molecule belonging to the category of steroidal anti-inflammatories, which may exist in insoluble or soluble lipophilic form, such as phosphate ester, a precursor of dexamethasone. In the present composition and mixture, for example in otological drops, dexamethasone is used as the phosphate ester, selected from sodium phosphate ester or disodium phosphate ester [2-[(8S,9R,10S,11S,13S,14S,16R,17R)-9-fluoro-11,17-di-hydroxy-10,13,16-trimethyl-3-oxo-6,7,8,11,12,14,15,16-octahydrocyclopenta[a]phenanthren-17-yl]-2-oxoethyl] phosphate. The structural formula of dexamethasone sodium phosphate is shown in Figure 2, in particular of dexamethasone disodium phosphate ester.

Besides levofloxacin and dexamethasone, the composition of the present invention also comprises a conservative selected from among the common classes or in use, in particular quaternary ammonium salts whose preferred and most widely used is benzalkonium chloride and a buffer system such as phosphate, citrate, borate buffers or combination thereof, capable of stabilising the pH value of the composition at a value around neutral pH 7, comprised from 5.5 to 8, preferably comprised from 6.7 to 7.8, even more preferably comprised between 7.0 to 7.4, for example being of 6.8; 6.9; 7.0; 7.1; 7.2;7.3;7.4.

It should be observed that the pH values specified in this description are to be understood as having a tolerance (±) - with respect to the specified pH value - of about 0.05, preferably with a tolerance (±) of about 0.03, more preferably with a tolerance (±) of about 0.02, even more preferably with a tolerance (±) of about 0.01. All pH values of the composition considered herein were measured at 20°C.

The present mixture (i) is in aqueous solution. More preferably, the water used in such mixture is distilled water or water for injection.

Although from the prior art (Robertson et al., 2005) levofloxacin has been known to have a solubility in water lower than 2% despite extremely long dissolution times, for example equal to a few hours, the Applicant surprisingly discovered that a suitable selection of the pH value of the composition allows to obtain several extremely advantageous technical effects.

First and foremost, in the present composition, for the first time levofloxacin is dissolved or solubilised in solution (that is, it is not dispersed or suspended) and, therefore, the composition does not have a precipitate or parts insoluble in solution at an amount sufficiently high to be therapeutically effective.

Similarly, at a pH value comprised from 5.5 to 8.0 (preferably comprised from 6.7 to 7.8, more preferably comprised from 7.0 to 7.4, it is also possible to obtain an equally satisfactory dissolution of dexamethasone.

Furthermore, a pH value comprised from 5.5 to 8.0 (preferably comprised from 6.7 to 7.8, more preferably comprised from 7.0 to 7.4) allows to obtain an optimal long-term stability up to 18 months and, not less important, a high tolerability with the outer ear, or with the middle ear, depending on the anatomical region of use of the present composition.

Dexamethasone in the dexamethasone phosphate ester form is a molecule soluble at certain pH values. On the other hand, levofloxacin (shown in Figure 1) is in zwitterionic form, that is it is an inner salt containing an anionic part and a cationic part, with a pKa1: 5.6; pKa2: 7.9, which means that the anionic, cationic, zwitterionic forms of the molecule coexist in solution form. The balance between the various forms of levofloxacin changes depending on the pH of the solution, which should preferably remain between 5.5 and 8 in order to stabilise the dissolution of both active ingredients.

The concentration of levofloxacin, for example levofloxacin hemihydrate, in the co-presence of dexamethasone, is comprised from 0.05 mg/ml to 20 mg/ml, preferably from 0.05 mg/ml to 15 mg/ml, 0.05 mg/ml to 10 mg/ml of composition in liquid solution, preferably it is comprised from 0.5 mg/ml to 15 mg/ml, or from 0.5 mg/ml to 12 mg/ml, or from 0.5 mg/ml to 8 mg/ml of composition in liquid solution, even more preferably it is comprised from 1 mg/ml to 7.5 mg/ml of composition in liquid solution, further preferably from 3 mg/ml to 6.5 mg/ml of composition in liquid solution, for example 1 mg/ml, 1.25 mg/ml, 2 mg/ml, 3 mg/ml, 3.5 mg/ml, 4.0 mg/ml, 4.5 mg/ml, 5.0 mg/ml, 5.5 mg/ml, 6.0 mg/ml, 7 mg/ml, 8 mg/ml, 9 mg/ml, 10 mg/ml, 11 mg/ml, or 12 mg/ml. These are levofloxacin values in combination (in the presence) with dexamethasone.

The concentration (mg/ml) of dexamethasone present in the composition and mixture with levofloxacin such as dexamethasone sodium phosphate ester (Figure 2) - or dexamethasone disodium phosphate ester, is comprised from 0.01 mg/ml to 5 mg/ml of composition in liquid solution, preferably it is comprised from 0.5 mg/ml to 3 mg/ml of composition in liquid solution, even more preferably it is comprised from 0.7 mg/ml to 2 mg/ml of composition in liquid solution, for example 0.75 mg/ml, 0.85 mg/ml, 0.95 mg/ml, 1.0 mg/ml, 1.1 mg/ml, 1.2 mg/ml, 1.3 mg/ml, 1.4 mg/ml, 1.5 mg/ml, or 2.0 mg/ml.

The otologic composition in solution of the present invention further comprises one or more other substances selected from among the group comprising or, alternatively, consisting of sodium phosphate, sodium phosphate monobasic monohydrate, sodium phosphate dibasic, sodium phosphate dibasic dodecahydrate, sodium citrate, sodium chloride, boric acid and/or borates, benzalkonium chloride, sodium hyaluronate or hyaluronic acid, 1 N NaOH for adjusting the pH to a value comprised from 6.7 to 7.8, preferably 7.0 to 7.4, for example 7.2, and water (distilled water or water for injection). Benzalkonium chloride acts as a preservative. In the composition of the present invention, benzalkonium chloride does not act as or does not serve the function of a surfactant or suspending agent.

A preferred embodiment refers to the compositions as reported in examples 1, 2, 3 and 4 for use in a treatment method which provides for the administration of the composition shown in Table 1, or of the composition shown in Table 3, or of the composition shown in Table 4, or of the composition shown in Table 5

### EXAMPLE 1

**Table 1**

| Components | Quantity (mg/ml) | Function |
|---|---|---|
| Levofloxacin hemihydrate | 5.12 (equivalent to 5 mg/ml levofloxacin) | Active |
| Dexamethasone sodium phosphate | 1.32 (equivalent to 1 mg/ml dexamethasone) | Active |
| Sodium phosphate monobasic monohydrate | 1.47 | Buffer agent |
| Dibasic sodium phosphate dodecahydrate | 10.0 | Buffer agent |
| Sodium citrate | 21.0 | Buffer agent |
| Benzalkonium chloride 50 % solution | 0.10 | Preservative |
| 1N NaOH | q.s. at pH 7.2 | PH modifier |
| Distilled water | q.s. at 1 ml | Diluent |

The composition of example 1 in Table 1 is a transparent clear green-yellow solution devoid of precipitate or suspended solid particles insoluble in solution at pH 7.2, and it has the main physical chemical specifications reported in Table 2.

**Table 2**

| Appearance | Clear solution |
|---|---|
| Colour of the solution | ≤ GY3 |
| pH | 7.2 (± 0.2) |
| Osmolarity | 300 mOsm/Kg (± 30) |

Forming an object of the present invention is a process for the preparation of the present composition, preferably in pharmaceutical form as otological drops, by means of methods, apparatus and instruments known to the man skilled in the art of sterile otological solutions.

The otologic composition subject of the present invention is a composition in sterile solution.

The process for preparing the composition of the present invention comprises adding and dissolving the components of Example 1 in water for injection in a reduced contamination and controlled area at a room temperature of around 20°C.

The aforementioned dissolution step is followed by an aseptic filtration step using a sterilising filter having a porosity of about 0.2 µm capable of retaining all microorganisms.

The aforementioned filtration step is followed by a step of filling/sealing the composition in the form of a sterile solution in pre-sterilised otological containers (in particular: containers for eye drops); said containers being provided with a dropper device and a cap closure device. The filling/sealing step is carried out under almost no contamination conditions, so-called class A area.

The composition in the form of a solution is filtered under sterile conditions and packaged in a plastic (primary) container made of opaque polyethylene, since levofloxacin is photosensitive. Said container is provided with a dropper which dispenses 30 microliters (0.03 ml) per drop.

The primary container of the eye drops solution is a light-opaque plastic bottle, conveniently made of low-density polyethylene (LDPE) containing 50 microliters of solution, provided with an opaque LDPE dropper and a high-density polyethylene (HDPE) screw cap.

Other embodiments of the composition of the present invention are described in example 2 (Table 3), example 3 (Table 4) and example 4 (Table 5) below.

### EXAMPLE 2 MULTI-DOSE FORMULA WITHOUT PRESERVATIVE

5 ml (from 3 to 10 ml) of solution having the following composition:

**Table 3**

| Components | Quantity (mg/ml) | Function |
|---|---|---|
| Levofloxacin hemihydrate | 5.12 (equivalent to 5 mg/ml levofloxacin) | Active |
| Dexamethasone sodium phosphate | 1.32 (equivalent to 1 mg/ml dexamethasone) | Active |
| Sodium phosphate monobasic monohydrate | 1.47 | Buffer agent |
| Dibasic sodium phosphate dodecahydrate | 10.0 | Buffer agent |
| Sodium citrate | 21.0 | Buffer agent |
| 1N NaOH | q.s. at pH 7.2 | PH modifier |
| Distilled water | q.s. at 1 ml | Diluent |

### EXAMPLE 3 SINGLE-DOSE FORMULA WITHOUT PRESERVATIVE

A volume of 0. 4 ml (from 0.1 to 1 ml) of solution having the following composition:

**Table 4**

| Components | Quantity (mg/ml) | Function |
|---|---|---|
| Levofloxacin hemihydrate | 5.12 (equivalent to 5 mg/ml levofloxacin) | Active |
| Dexamethasone sodium phosphate | 1.32 (equivalent to 1 mg/ml dexamethasone) | Active |
| Sodium phosphate monobasic monohydrate | 1.47 | Buffer agent |
| Dibasic sodium phosphate dodecahydrate | 10.0 | Buffer agent |
| Sodium citrate | 21.0 | Buffer agent |
| 1N NaOH | q.s. at pH 7.2 | PH modifier |
| Distilled water | q.s. at 1 ml | Diluent |

### EXAMPLE 4 VISCOSIFIED FORMULA FOR BETTER BIOADHESION:

**Table 5**

| Components | Quantity (mg/ml) | Function |
|---|---|---|
| Levofloxacin hemihydrate | 5.12 (equivalent to 5 mg/ml levofloxacin) | Active |
| Dexamethasone sodium phosphate | 1.32 (equivalent to 1 mg/ml dexamethasone) | Active |
| Sodium phosphate monobasic monohydrate | 1.47 | Buffer agent |
| Dibasic sodium phosphate dodecahydrate | 10.0 | Buffer agent |
| Sodium citrate | 21.0 | Buffer agent |
| 1N NaOH | q.s. at pH 7.2 | PH modifier |
| Sodium hyaluronate | 0.3% | Viscosifier |
| Distilled water | q.s. at 1 ml | Diluent |

### EXAMPLE 5 ACCELERATED AND INTERMEDIATE STABILITY TEST

The solutions of Examples 1-4 of the present invention were all tested, with mutually corresponding outcomes. However, for the sake of simplicity, we will only report a study carried out with the solution of example 1.

The batches produced were subjected to an accelerated stability study and in tropical climatic conditions, the most severe of which is zone IVb.

The climatic stability zones for drugs, long-term test conditions and the accelerated and intermediate test conditions identified by the harmonised guideline of the International Harmonization testing ICH Q1A and Q1F are respectively reported in Tables 6, 7, 8 below.

**Table 6: ICH stability zones**

| Zones | Climate type |
|---|---|
| Zone I | Temperate zone |
| Zone II | Mediterranean / subtropical zone |
| Zone III | Hot and dry zone |
| Zone IV | Hot humid / tropical zone |
| Zone IVb | ASEAN test conditions higher heat/humidity |

**Table 7: Long-term test conditions**

| Climatic zone | Temperature | Relative humidity (RH) | Minimum duration |
|---|---|---|---|
| Zone I | 21°C ± 2°C | 45% ± 5% | 12 months |
| Zone II | 25°C ± 2°C | 60% ± 5% | 12 months |
| Zone III | 30°C ± 2°C | 35% ± 5% | 12 months |
| Zone IV | 30°C ± 2°C | 65% ± 5% | 12 months |
| Zone IVb | 30°C ± 2°C | 75% ± 5% | 12 months |
| Refrigerated | 5°C ± 3°C | No humidity | 12 months |
| Frozen | -15°C ± 5°C | No humidity | 12 months |

**Table 8: Accelerated and intermediate test conditions**

| Climatic zone | Temperature | Relative humidity (RH) | Minimum duration |
|---|---|---|---|
| Accelerated environment | 40°C ± 2°C | 75% ± 5% | 6 months |
| Accelerated refrigerated | 25°C ± 2°C | 60% ± 5% | 6 months |
| Accelerated frozen | 5°C ± 3°C | No humidity | 6 months |
| Intermediate | 30°C ± 2°C | 65% ± 5% | 6 months |

Previously, the long-term test conditions (Table 7) and the accelerated and intermediate test conditions (Table 8) were reported, given that accelerated and intermediate stability are usually predictive and prodromal than the long-term stability.

On the other hand, reported on Table 9 below are the product specifications proposed at the time of preparation of the composition (*Release specifications*) and at the end of validity (*Shelf-life specifications*)*.*

**Table 9: Proposed product specifications**

| Parameters | | Release specifications | Shelf-life specifications |
|---|---|---|---|
| Appearance | | Clear green-yellow solution | Clear green-yellow solution |
| Colour | | ≤ GY3 | ≤ GY3 |
| pH | | 7.0-7.4 | 6.8-7.6 |
| Osmolality | | 270 - 330 (mOsm /Kg) | 270 - 330 (mOsm /Kg) |
| Levofloxacin identification | | Positive | Positive |
| Dexamethasone identification | | Positive | Positive |
| Benzalkonium chloride identification | | Positive | Positive |
| Levofloxacin assay | | 95 - 105 % of the indication on the label | 95 - 110% of the indication on the label |
| Dexamethasone sodium phosphate assay | | 95 - 105 % of the indication on the label (LC) | 95 - 110% of the indication on the label (LC) |
| Substances bound to levofloxacin (%): | | | |
| | - N-desmethyl levofloxacin | ≤ 0.3 % | ≤ 1.0 % |
| | - Levofloxacin N-oxide | ≤ 0.3 % | ≤ 1.0 % |
| | - Diamino derivative | ≤ 0.3 % | ≤ 1.0 % |
| Every unknown impurity | | ≤ 0.2% | ≤ 0.5% |
| Total impurities | | ≤ 1.0 % | ≤ 2.0 % |
| Substances bound to dexamethasone sodium phosphate (%) | | | |
| | - dexamethasone (imp A) | ≤ 0.5 % | ≤ 2.0 % |
| | - D Homo A (Imp. C) | ≤ 0.3 % | ≤ 1.0 % |
| | - D Homo B (Imp. D) | ≤ 0.3 % | ≤ 1.0 % |
| | - Impurity G | ≤ 0.2 % | ≤ 0.2 % |
| | - Every unknown impurity | ≤ 0.2 % | ≤ 1.0 % |
| Total impurities | | ≤ 1.0 % | ≤ 3.0 % |
| Benzalkonium chloride assay | | 90 - 110 % LC | 90 - 110% LC |
| Sterility | | Sterile | Sterile |
| Volume filled | | ≥ 5ml | ≥ 5ml |

The solution of example 1 was subjected to an accelerated stability test under the following conditions: + 30°C ± 2°C / 75% ± 5% RH for the long-term stability test, whose results of are reported in Table 10; + 40°C ± 2°C / 75% ± 5% RH for the accelerated stability test, whose results are reported in Table 11. Tests were also conducted relating to freezing-thawing cycles (Table 12; 3 48-hour cycles between - 20°C and + 25°C) and to the thermal excursion (Table 13; 3 48-hour cycles between - 20°C and + 40°C).

**Table 10: + 30°C ± 2°C / 75% ± 5% RH; long-term**

| Parameters | | Release specifications | T = 0 months | T = 6 months |
|---|---|---|---|---|
| Appearance | | Clear green-yellow solution; devoid of particulates | Clear green-yellow solution; devoid of particulates | Clear green-yellow solution; devoid of particulates |
| Colour | | ≤ GY3 | < GY3 | < GY3 |
| pH | | 6.8-7.6 | 7.2 | 7.2 |
| Osmolality | | 270 - 330 (mOsm /Kg) | 301 | 301 |
| Volume filled | | ≥ 5.0 ml | 5.3 ml | 5.3 ml |
| Sterility | | Sterile | Sterile | |
| Benzalkonium chloride assay (g/100 ml) | | 0.0045% - 0.0055% w/v (90.0% - 110.0% LC) | 0.0050 (100.0%) | n.d. |
| Levofloxacin assay | | 0.450% - 0.550% w/v (90.0% - 110.0% LC) | 0.513 (102.6%) | 0.516 (103.2%) |
| | N-desmethyl levofloxacin | ≤ 1.0% | 0.1 | 0.1 |
| | levofloxacin N-oxide | ≤ 1.0% | <LOQ*) | 0.1 |
| | Diamino derivative | ≤ 1.0% | <LOQ | <LOQ |
| | Every unknown impurity | ≤ 0.5% | <LOQ | <LOQ |
| | Total impurities | ≤ 2.0% | 0.1 | 0.2 |
| Dexamethasone sodium phosphate assay | | 0.119% - 0.145% w/v (90.0% - 110.0% LC) | 0.135 (102.3%) | 0.135 (102.3%) |
| | Dexamethasone (Imp. A) | ≤ 2.0% | <LOQ | 0.4 |
| | D Homo A (Imp. C) | ≤ 1.0% | <LOQ | 0.1 |
| | D Homo B (Imp. D) | ≤ 1.0% | <LOQ | <LOQ |
| | Impurity G | ≤ 0.2% | 0.1 | 0.1 |
| | Every unknown impurity | ≤ 1.0% | <LOQ | <LOQ |
| | Total impurities | ≤ 3.0% | 0.1 | 0.6 |

| | | | | |
|---|---|---|---|---|
| * = less than the quantification limit | | | | |

**Table 11: + 40°C±2°C/75%±5% RH; accelerated (release specifications identical to those reported in Table 10).**

| Parameters | | T = 0 months | T = 1 month | T = 6 months |
|---|---|---|---|---|
| Appearance | | Clear green-yellow solution; devoid of particulates | Clear green-yellow solution; devoid of particulates | Clear green-yellow solution; devoid of particulates |
| Colour | | < GY3 | < GY3 | < GY3 |
| pH | | 7.2 | 7.2 | 7.2 |
| Osmolality | | 301 | 288 | 310 |
| Volume filled | | 5.3 ml | 5.3 ml | 5.1 ml |
| Sterility | | n.d. | n.d. | Sterile |
| Benzalkonium chloride assay (g/100 ml) | | 0.0050 (100.0%) | 0.0049 (98.0%) | n.d. |
| Levofloxacin assay | | 0.513 (102.6%) | 0.520 (103.9%) | 0.530 (106.0%) |
| | N-desmethyl levofloxacin | 0.1 | <LOQ | 0.1 |
| | levofloxacin N-oxide | <LOQ | 0.1 | 0.3 |
| | Diamino derivative | <LOQ | <LOQ | <LOQ |
| | Every unknown impurity | <LOQ | <LOQ | <LOQ |
| | Total impurities | 0.1 | 0.1 | 0.4 |
| Dexamethasone sodium phosphate assay | | 0.135 (102.3%) | 0.135 (102.3%) | 0.135 (102.3%) |
| | Dexamethasone (Imp. A) | <LOQ | 0.4 | 1.5 |
| | D Homo A (Imp. C) | <LOQ | <LOQ | 0.2 |
| | D Homo B (Imp. D) | <LOQ | <LOQ | 0.1 |
| | Impurity G | 0.1 | <LOQ | 0.1 |
| | Every unknown impurity | <LOQ | <LOQ | 0.2 |
| | Total impurities | 0.1 | 0.4 | 2.2 |

**Table 12: freezing-thawing cycle results**

| Parameters | | Release specifications | T = 0 months | Third cycle |
|---|---|---|---|---|
| Appearance | | Clear green-yellow solution; devoid of particulates | Clear green-yellow solution; devoid of particulates | Clear green-yellow solution; devoid of particulates |
| Colour | | ≤ GY3 | < GY3 | < GY3 |
| pH | | 6.8-7.6 | 7.3 | 7.3 |
| Osmolality | | 270 - 330 (mOsm /Kg) | 281 | 297 |
| Benzalkonium chloride assay (g/100 ml) | | 0.0045% - 0.0055% w/v (90.0% - 110.0% LC) | 0.0048 (96.0%) | 0.0050 (100.0%) |
| Levofloxacin assay | | 0.450% - 0.550% w/v (90.0% - 110.0% LC) | 0.518 (103.6%) | 0.523 (104.5%) |
| | N-desmethyl levofloxacin | ≤ 1.0% | ≤ 0.05% | ≤ 0.05% |
| | levofloxacin N-oxide | ≤ 1.0% | ≤ 0.05% | ≤ 0.05% |
| | Diamino derivative | ≤ 1.0% | ≤ 0.05% | ≤ 0.05% |
| | Every unknown impurity | ≤ 0.5% | ≤ 0.05% | ≤ 0.05% |
| | Total impurities | ≤ 2.0% | ≤ 0.05% | ≤ 0.05% |
| Dexamethasone sodium phosphate assay | | 0.119% - 0.145% w/v (90.0% - 110.0% LC) | 0.135 (102.3%) | 0.134 (101.5%) |
| | Dexamethasone (Imp. A) | ≤ 2.0% | 0.2% | ≤ 0.05% |
| | D Homo A (Imp. C) | ≤ 1.0% | ≤ 0.05% | ≤ 0.05% |
| | D Homo B (Imp. D) | ≤ 1.0% | ≤ 0.05% | ≤ 0.05% |
| | Impurity G | ≤ 0.2% | ≤ 0.05% | ≤ 0.05% |
| | Every unknown impurity | ≤ 1.0% | RRT0.9=0.2% | ≤ 0.05% |
| | Total impurities | ≤ 3.0% | 0.4% | ≤ 0.05% |

**Table 13: thermal excursion results**

| Parameters | | Release specifications | T = 0 months | Third cycle |
|---|---|---|---|---|
| Appearance | | Clear green-yellow solution; devoid of particulates | Clear green-yellow solution; devoid of particulates | Clear green-yellow solution; devoid of particulates |
| Colour | | ≤ GY3 | < GY3 | < GY3 |
| pH | | 6.8-7.6 | 7.3 | 7.3 |
| Osmolality | | 270 - 330 (mOsm /Kg) | 281 | 298 |
| Benzalkonium chloride assay (g/100 ml) | | 0.0045% - 0.0055% w/v (90.0% - 110.0% LC) | 0.0048 (96.0%) | 0.0050 (100.0%) |
| Levofloxacin assay | | 0.450% - 0.550% w/v (90.0% - 110.0% LC) | 0.518 (103.6%) | 0.522 (104.4%) |
| | N-desmethyl levofloxacin | ≤ 1.0% | ≤ 0.05% | ≤ 0.05% |
| | levofloxacin N-oxide | ≤ 1.0% | ≤ 0.05% | ≤ 0.05% |
| | Diamino derivative | ≤ 1.0% | ≤ 0.05% | ≤ 0.05% |
| | Every unknown impurity | ≤ 0.5% | ≤ 0.05% | ≤ 0.05% |
| | Total impurities | ≤ 2.0% | ≤ 0.05% | ≤ 0.05% |
| Dexamethasone sodium phosphate assay | | 0.119% - 0.145% w/v (90.0% - 110.0% LC) | 0.135 (102.3%) | 0.135 (102.3%) |
| | Dexamethasone (Imp. A) | ≤ 2.0% | 0.2% | ≤ 0.05% |
| | D Homo A (Imp. C) | ≤ 1.0% | ≤ 0.05% | ≤ 0.05% |
| | D Homo B (Imp. D) | ≤ 1.0% | ≤ 0.05% | ≤ 0.05% |
| | Impurity G | ≤ 0.2% | ≤ 0.05% | ≤ 0.05% |
| | Every unknown impurity | ≤ 1.0% | RRT0.9=0.2% | ≤ 0.05% |
| | Total impurities | ≤ 3.0% | 0.4% | ≤ 0.05% |

The study was conducted and completed successfully. The study surprisingly revealed that the composition in solution reported in example 1 shows a markedly better stability than prior art suspensions (Cilodex^{®} and Tobradex^{®}).

As a matter fact, the composition subject of the present invention has the ability not to deteriorate even if stored at temperatures higher than room temperature (at 30°C as in Table 10, or at 40°C as in Table 11) for several months, currently up to 18, and at temperatures lower than the freezing temperature (Table 12), also following repeated freezing and thawing cycles.

In this regard, but without intending to provide any scientific explanation of the phenomenon, it is believed that such greater stability is possible thanks to the dissolution (and not to the dispersion or suspension) of the two active ingredients, in particular dexamethasone sodium phosphate, anc levofloxacin, in particular levofloxacin hemihydrate, in the composition.

Thus, according to the invention, the composition of the present invention is devoid of surfactant anc suspending substances, and, as such, it is devoid of TWEEN-80 (emulsifier) and devoid of PEG-400. Furthermore, the present composition may also be devoid of a vitamin E tocopheryl or tocopherol derivative.

Thus, possible cooling/freezing and subsequent heating of the present composition could also give rise to phenomena relating to the precipitation and re-dissolution of the active ingredients contained therein, but they do not cause irreversible and destructive alterations to the composition, capable of altering its efficacy.

Another highly advantageous aspect of an otologic composition in aqueous solution, comprising the mixture in aqueous solution, instead of the suspensions according to the prior art, lies in the fact that otitis with STMP can be treated with a preparation which does not entail the presence or the deposit of solid residues. Advantageously, the otologic composition in aqueous solution of the present invention finds valid application for use in a method for the treatment of bacterial ear infections, preferably for the treatment of acute otitis externa (AOE) and acute otitis media (AOM).

In one embodiment, this otological composition in aqueous solution, for use in a method of treatment of bacterial ear infections, for use in a method of treatment of acute otitis externa (AOE) finds a valid application when the otological bacterial infections are caused, derived or originated preferably from *Streptococcus pyogenes, Staphylococcus aureus, Staphylococcus epidermidis, Staphylococcus haemolyticus, Pseudomonas aeruginosa, Enterococcus faecalis* and *Enterobacter cloacae.*

In one embodiment, this otological composition in aqueous solution, for use in a method of treatment of bacterial ear infections, for use in a method of treatment of acute otitis media (AOM) finds a valid application when the otological bacterial infections are caused, derived or originated preferably by *Streptococcus pneumoniae, Haemophilus influenzae* and *Moraxella catarrhalis.*

As a matter of fact, at present, otitis externa is treated through a topical administration of suspensions or dispersions; however, such treatment is ineffective. On the other hand, the treatment of otitis media, which are inaccessible from the outside, is managed only with a systemic oral administration of very high doses of antibiotic, doses which are much higher than the amount of antibiotic used in otological drops for local use.

The present composition comprising the mixture in solution therefore opens a new opportunity for the treatment of otitis externa, guaranteeing the non-precipitation of any solid deposits present in other known formulations in suspension or dispersion, and otitis media through the local topical route, thanks to the possible transit of the mixture in solution through the tympanic membrane (with or without STMP), even opening up to the possibility of exclusive topical treatment instead of the currently administered systemic treatments.

Therefore, the composition of the present invention finds a valid application for use in a method for the treatment of otitis externa and otitis media preferably in subjects with spontaneous perforation of the tympanic membrane.

## Claims

1. An otologic composition in aqueous solution comprising a mixture in aqueous solution comprising, or alternatively, consisting of an antibiotic, which is levofloxacin or a salt thereof or a hydrate thereof or a hemihydrate thereof and a corticosteroid, which is dexamethasone sodium phosphate selected from dexamethasone sodium phosphate ester or dexamethasone disodium phosphate ester dissolved in the aqueous solution, and optionally, one or more pharmaceutical grade additives and/or excipients; wherein said composition is devoid of surfactant and suspending substances; wherein said composition has a pH value comprised from 5.5 to 8.0; said composition being for use in a method for the treatment of otological bacterial infections.

2. The composition for use according to the preceding claim, wherein said pH value is comprised from 6.7 to 7.8, preferably comprised from 7.0 to 7.4, even more preferably being 7.2.

3. The composition for use according to any one of the preceding claims, wherein said levofloxacin or a salt thereof or a hemihydrate thereof or a hydrate thereof is present in said composition in solution at a concentration comprised from 0.05 mg/ml to 20 mg/ml, preferably from 0.05 mg/ml to 15 mg/ml, 0.05 mg/ml to 10 mg/ml of composition in liquid solution, preferably it is comprised from 0.5 mg/ml to 15 mg/ml, or from 0.5 mg/ml to 12 mg/ml, or from 0.5 mg/ml to 8 mg/ml of composition in liquid solution, even more preferably it is comprised from 1 mg/ml to 7.5 mg/ml of composition in liquid solution, further preferably from 3 mg/ml to 6.5 mg/ml of composition in liquid solution, for example 1 mg/ml, 1.25 mg/ml, 2 mg/ml, 3 mg/ml, 3.5 mg/ml, 4.0 mg/ml, 4.5 mg/ml, 5.0 mg/ml, 5.5 mg/ml, 6.0 mg/ml, 7 mg/ml, 8 mg/ml, 9 mg/ml, 10 mg/ml, 11 mg/ml, or 12 mg/ml.

4. The composition for use according to any one of the preceding claims, wherein said dexamethasone is present in said composition in solution at a concentration comprised from 0.01 mg/ml to 5 mg/ml of composition in liquid solution, preferably it is comprised from 0.5 mg/ml to 3 mg/ml of composition in liquid solution, even more preferably it is comprised from 0.7 mg/ml to 2 mg/ml of composition in liquid solution, for example 0.75 mg/ml, 0.85 mg/ml, 0.95 mg/ml, 1.0 mg/ml, 1.1 mg/ml, 1.2 mg/ml, 1.3 mg/ml, 1.4 mg/ml, 1.5 mg/ml, or 2.0 mg/ml.

5. The composition for use according to any one of the preceding claims, wherein said mixture is in aqueous solution, preferably distilled water or water for injection.

6. The composition for use according to any one of the preceding claims, wherein said composition is for use in a method for the treatment of otological bacterial infections in subjects of any age.

7. The composition for use according to any one of the preceding claims, wherein said composition is for use in a method for the treatment of acute otitis externa and acute otitis media.

8. The composition for use according to any one of the preceding claims, wherein said composition is for use in a method for the treatment of acute otitis interna or for the treatment of acute otitis media, preferably in subjects with a spontaneous perforation of the tympanic membrane, more preferably in substitution of the systemic oral treatment.

9. The composition for use according to any one of the preceding claims, wherein said otological composition in aqueous solution, for use in a method of treatment of bacterial ear infections, for use in a method of treatment of acute otitis externa finds a valid application when the otological bacterial infections are caused, derived or originated preferably from *Streptococcus pyogenes, Staphylococcus aureus, Staphylococcus epidermidis, Staphylococcus haemolyticus, Pseudomonas aeruginosa, Enterococcus faecalis* and *Enterobacter cloacae.*

10. The composition for use according to any one of the preceding claims, wherein said otological composition in aqueous solution, for use in a method of treatment of bacterial ear infections, for use in a method of treatment of acute otitis media finds a valid application when the otological bacterial infections are caused, derived or originated preferably by *Streptococcus pneumoniae, Haemophilus influenzae* and *Moraxella catarrhalis.*

11. The composition for use according to any one of the preceding claims, **characterised in that** it is a composition for topical otological use.

## Patentansprüche

1. Otologische Zusammensetzung in wässriger Lösung, umfassend eine Mischung in wässriger Lösung, umfassend oder alternativ bestehend aus einem Antibiotikum, das Levofloxacin oder ein Salz davon oder ein Hydrat davon oder ein Halbhydrat davon ist, und einem Corticosteroid, das Dexamethason-Natriumphosphat ist, ausgewählt aus Dexamethason-Natriumphosphatester oder Dexamethason-Dinatriumphosphatester, gelöst in der wässrigen Lösung, und gegebenenfalls einem oder mehreren pharmazeutischen Zusatzstoffen und/oder Hilfsstoffen; wobei die Zusammensetzung frei von oberflächenaktiven und suspendierenden Substanzen ist; wobei die Zusammensetzung einen pH-Wert im Bereich von 5,5 bis 8,0 aufweist; wobei die Zusammensetzung zur Verwendung in einem Verfahren zur Behandlung otologischer bakterieller Infektionen dient.

2. Zusammensetzung zur Verwendung nach dem vorhergehenden Anspruch, wobei der pH-Wert im Bereich von 6,7 bis 7,8, vorzugsweise im Bereich von 7,0 bis 7,4, noch bevorzugter bei 7,2 liegt.

3. Zusammensetzung zur Verwendung nach einem der vorhergehenden Ansprüche, wobei das Levofloxacin oder ein Salz davon oder ein Halbhydrat davon oder ein Hydrat davon in der Zusammensetzung in Lösung in einer Konzentration im Bereich von 0,05 mg/ml bis 20 mg/ml, vorzugsweise von 0,05 mg/ml bis 15 mg/ml, 0,05 mg/ml bis 10 mg/ml der Zusammensetzung in flüssiger Lösung vorliegt, es vorzugsweise im Bereich von 0,5 mg/ml bis 15 mg/ml oder von 0,5 mg/ml bis 12 mg/ml oder von 0,5 mg/ml bis 8 mg/ml der Zusammensetzung in flüssiger Lösung liegt, es noch bevorzugter im Bereich von 1 mg/ml bis 7,5 mg/ml der Zusammensetzung in flüssiger Lösung liegt, weiter bevorzugt von 3 mg/ml bis 6,5 mg/ml der Zusammensetzung in flüssiger Lösung, zum Beispiel 1 mg/ml, 1,25 mg/ml, 2 mg/ml, 3 mg/ml, 3,5 mg/ml, 4,0 mg/ml, 4,5 mg/ml, 5,0 mg/ml, 5,5 mg/ml, 6,0 mg/ml, 7 mg/ml, 8 mg/ml, 9 mg/ml, 10 mg/ml, 11 mg/ml oder 12 mg/ml.

4. Zusammensetzung zur Verwendung nach einem der vorhergehenden Ansprüche, wobei das Dexamethason in der Zusammensetzung in Lösung in einer Konzentration im Bereich von 0,01 mg/ml bis 5 mg/ml der Zusammensetzung in flüssiger Lösung liegt, es vorzugsweise im Bereich von 0,5 mg/ml bis 3 mg/ml der Zusammensetzung in flüssiger Lösung liegt, es noch bevorzugter im Bereich von 0,7 mg/ml bis 2 mg/ml der Zusammensetzung in flüssiger Lösung liegt, zum Beispiel 0,75 mg/ml, 0,85 mg/ml, 0,95 mg/ml, 1,0 mg/ml, 1,1 mg/ml, 1,2 mg/ml, 1,3 mg/ml, 1,4 mg/ml, 1,5 mg/ml oder 2,0 mg/ml.

5. Zusammensetzung zur Verwendung nach einem der vorhergehenden Ansprüche, wobei die Mischung in wässriger Lösung, vorzugsweise destilliertem Wasser oder Wasser für Injektionszwecke, ist.

6. Zusammensetzung zur Verwendung nach einem der vorhergehenden Ansprüche, wobei die Zusammensetzung zur Verwendung in einem Verfahren zur Behandlung otologischer bakterieller Infektionen bei Probanden jeden Alters dient.

7. Zusammensetzung zur Verwendung nach einem der vorhergehenden Ansprüche, wobei die Zusammensetzung zur Verwendung in einem Verfahren zur Behandlung von akuter Otitis externa und akuter Otitis media dient.

8. Zusammensetzung zur Verwendung nach einem der vorhergehenden Ansprüche, wobei die Zusammensetzung zur Verwendung in einem Verfahren zur Behandlung von akuter Otitis interna oder zur Behandlung von akuter Otitis media, vorzugsweise bei Probanden mit einer spontanen Perforation des Trommelfells, bevorzugter in Substitution der systemischen oralen Behandlung, dient.

9. Zusammensetzung zur Verwendung nach einem der vorhergehenden Ansprüche, wobei die otologische Zusammensetzung in wässriger Lösung zur Verwendung in einem Verfahren zur Behandlung von bakteriellen Ohrinfektionen zur Verwendung in einem Verfahren zur Behandlung von akuter Otitis externa eine fundierte Anwendung findet, wenn die otologischen bakteriellen Infektionen vorzugsweise durch *Streptococcus pyogenes, Staphylococcus aureus, Staphylococcus epidermidis, Staphylococcus haemolyticus, Pseudomonas aeruginosa, Enterococcus faecalis* und *Enterobacter cloacae* verursacht, von diesen abgeleitet werden oder aus diesen stammen.

10. Zusammensetzung zur Verwendung nach einem der vorhergehenden Ansprüche, wobei die otologische Zusammensetzung in wässriger Lösung zur Verwendung in einem Verfahren zur Behandlung von bakteriellen Ohrinfektionen zur Verwendung in einem Verfahren zur Behandlung von akuter Otitis media eine fundierte Anwendung findet, wenn die otologischen bakteriellen Infektionen vorzugsweise durch *Streptococcus pneumoniae, Haemophilus influenzae* und *Moraxella catarrhalis* verursacht, von diesen abgeleitet werden oder aus diesen stammen.

11. Zusammensetzung zur Verwendung nach einem der vorhergehenden Ansprüche, **dadurch gekennzeichnet, dass** es sich um eine Zusammensetzung zur topischen otologischen Verwendung handelt.

## Revendications

1. Composition otologique en solution aqueuse comprenant un mélange en solution aqueuse comprenant, ou alternativement, constituée d'un antibiotique, qui est la lévofloxacine ou un de ses sels ou un de ses hydrates ou un de ses semi-hydrates et un corticostéroïde, qui est le phosphate sodique de dexaméthasone choisi parmi l'ester de phosphate sodique de dexaméthasone ou l'ester de phosphate disodique de dexaméthasone dissous dans la solution aqueuse, et éventuellement, un ou plusieurs additifs et/ou excipients de qualité pharmaceutique ; dans laquelle ladite composition est dépourvue de substances tensioactives et suspensives ; dans laquelle ladite composition a une valeur de pH comprise entre 5,5 et 8,0 ; ladite composition étant destinée à être utilisée dans une méthode de traitement des infections bactériennes otologiques.

2. Composition à utiliser selon la revendication précédente, dans laquelle la valeur de pH est comprise entre 6,7 et 7,8, de préférence comprise entre 7,0 et 7,4, plus préférablement encore est de 7,2.

3. Composition à utiliser selon l'une quelconque des revendications précédentes, dans laquelle ladite lévofloxacine, ou un de ses sels ou un de ses semi-hydrates ou un de ses hydrates, est présente dans ladite composition en solution à une concentration comprise entre 0,05 et 20 mg/ml, de préférence entre 0,05 et 15 mg/ml, 0,05 à 10 mg/ml de composition en solution liquide, de préférence comprise entre 0,5 et 15 mg/ml, ou entre 0,5 et 12 mg/ml, ou de 0,5 à 8 mg/ml de composition en solution liquide, plus préférablement comprise entre 1 et 7,5 mg/ml de composition en solution liquide, plus préférablement encore de 3 à 6,5 mg/ml de composition en solution liquide, par exemple 1 mg/ml, 1,25 mg/ml, 2 mg/ml, 3 mg/ml, 3,5 mg/ml, 4,0 mg/ml, 4,5 mg/ml, 5,0 mg/ml, 5,5 mg/ml, 6,0 mg/ml, 7 mg/ml, 8 mg/ml, 9 mg/ml, 10 mg/ml, 11 mg/ml ou 12 mg/ml.

4. Composition à utiliser selon l'une quelconque des revendications précédentes, dans laquelle ladite dexaméthasone est présente dans ladite composition en solution à une concentration comprise entre 0,01 et 5 mg/ml de composition en solution liquide, de préférence comprise entre 0,5 et 3 mg/ml de composition en solution liquide, plus préférablement encore de 0,7 à 2 mg/ml de composition en solution liquide, par exemple 0,75 mg/ml, 0,85 mg/ml, 0,95 mg/ml, 1,0 mg/ml, 1,1 mg/ml, 1,2 mg/ml, 1,3 mg/ml, 1,4 mg/ml, 1,5 mg/ml, ou 2,0 mg/ml.

5. Composition à utiliser selon l'une quelconque des revendications précédentes, dans laquelle le mélange est en solution aqueuse, de préférence de l'eau distillée ou de l'eau pour injection.

6. Composition à utiliser selon l'une quelconque des revendications précédentes, dans laquelle ladite composition est destinée à être utilisée dans une méthode de traitement des infections bactériennes otologiques chez des sujets de tout âge.

7. Composition à utiliser selon l'une quelconque des revendications précédentes, dans laquelle ladite composition est destinée à être utilisée dans une méthode de traitement de l'otite externe aiguë et de l'otite moyenne aiguë.

8. Composition à utiliser selon l'une quelconque des revendications précédentes, dans laquelle ladite composition est destinée à être utilisée dans une méthode pour le traitement de l'otite interne aiguë ou pour le traitement de l'otite moyenne aiguë, de préférence chez les sujets présentant une perforation spontanée de la membrane tympanique, plus préférablement en substitution du traitement oral systémique.

9. Composition à utiliser selon l'une quelconque des revendications précédentes, dans laquelle ladite composition otologique en solution aqueuse, à utiliser dans une méthode de traitement des infections bactériennes de l'oreille, à utiliser dans une méthode de traitement de l'otite externe aiguë trouve une application valable lorsque les infections bactériennes otologiques sont, de préférence, causées par, dérivées ou originaires de *Streptococcus pyogenes, Staphylococcus aureus, Staphylococcus epidermidis, Staphylococcus haemolyticus, Pseudomonas aeruginosa, Enterococcus faecalis et Enterobacter cloacae.*

10. Composition à utiliser selon l'une quelconque des revendications précédentes, dans laquelle ladite composition otologique en solution aqueuse, à utiliser dans une méthode de traitement des infections bactériennes de l'oreille, à utiliser dans une méthode de traitement de l'otite moyenne aiguë, trouve une application valable lorsque les infections bactériennes otologiques sont, de préférence, causées par, dérivées ou originaires de *Streptococcus pneumoniae, Haemophilus influenzae* et *Moraxella catarrhalis.*

11. Composition à utiliser selon l'une quelconque des revendications précédentes, **caractérisée en ce qu'**il s'agit d'une composition à usage otologique topique.
